(19) Europäisches Patentamt
European Patent Office
Office européen des brevets

(11) Numéro de publication: **0 168 293 B1**

(12) **FASCICULE DE BREVET EUROPEEN**

(45) Date de publication du fascicule du brevet: **05.08.87**

(51) Int. Cl.[4]: **C 07 C 51/567**, C 07 C 53/18

(21) Numéro de dépôt: **85401140.0**

(22) Date de dépôt: **11.06.85**

(54) **Procédé de préparation d'anhydride trifluoracétique.**

(30) Priorité: **13.06.84 FR 8409188**

(43) Date de publication de la demande: **15.01.86 Bulletin 86/3**

(45) Mention de la délivrance du brevet: **05.08.87 Bulletin 87/32**

(84) Etats contractants désignés: **AT BE CH DE FR GB IT LI LU NL SE**

(56) Documents cités: **DE - A - 3 107 108**

(73) Titulaire: **RHONE-POULENC SPECIALITES CHIMIQUES, "Les Miroirs" 18, Avenue d'Alsace, F-92400 Courbevoie (FR)**

(72) Inventeur: **Amiet, Louis, 10, quai Saint-Vincent, F-69001 - Lyon (FR)**
Inventeur: **Disdier, Camille, 12, rue Barodet, F-69004 - Lyon (FR)**

(74) Mandataire: **Fabre, Madeleine-France et al, RHONE-POULENC INTERSERVICES Service Brevets Chimie 25, quai Paul Doumer, F-92408 Courbevoie Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne un procédé de préparation d'anhydride trifluoracétique. Elle concerne plus particulièrement un procédé de préparation d'anhydride trifluoracétique par échange entre l'acide trifluoracétique et un anhydride d'acide.

Il est connu de préparer l'anhydride trifluoracétique (Hudlicky, Chemistry of Organic Fluorine Compounds, 1976, p. 726) par réaction de l'acide trifluoracétique sur l'anhydride phosphorique. Cette méthode de préparation est très difficilement exploitable industriellement car la phase solide constituée de l'anhydride phosphorique a tendance à former des amalgames empêchant en fin de réaction la récupération totale de l'acide trifluoracétique non transformé. Or l'acide trifluoracétique est un produit très onéreux et toute perte est à éviter.

Il est aussi connu d'après la demande de brevet japonais n° 70.38523 de préparer l'anhydride trifluoracétique par réaction du chlorure de trifluoracétyle avec un sel alcalin ou alcalino-terreux de l'acide trifluoracétique à environ 50° C. Ce procédé est aussi très difficile à exploiter industriellement car le chlorure de trifluoracétyle n'est pas un produit disponible couramment sur le marché donc d'un prix très élevé; d'autre part il est gazeux et difficile à mettre en œuvre; en outre, l'anhydride trifluoracétique formé est difficilement séparable du chlorure de sodium produit secondairement.

Il est également connu d'après la demande de brevet européen publiée sous le n° 4641 de préparer des anhydrides d'acide de formule

$$R-CO-O-CO-R$$

dans laquelle R peut être $CF_3$ par condensation de l'acide RCOOH et de l'anhydride acétique sous pression atmosphérique. Cette demande de brevet ne décrit aucun exemple de préparation de l'anhydride trifluoracétique. En effet, lorsque l'on procède selon ce procédé en utilisant comme acide l'acide trifluoracétique, on n'obtient pratiquement pas d'anhydride trifluoracétique car les proportions stœchiométriques des produits de départ employées ne permettent pas de déplacer l'anhydride mixte formé $CF_3COOCOCH_3$ qui est extrêmement stable.

Devant tous ces produits techniques et économiques, il a été nécessaire de trouver un procédé économique, facile à mettre en œuvre pour obtenir l'anhydride trifluoracétique. La présente invention permet d'atteindre cet objectif et a pour objet un procédé de préparation d'anhydride trifluoracétique caractérisé par le fait que l'on fait réagir l'acide trifluoracétique avec l'anhydride d'un acide halogéné.

L'anhydride d'acide α halogéné répond à la formule générale (I) suivante:

$$[Y_1\ (CY_2Y_3)_n-CO]_2O \qquad (I)$$

dans laquelle:

– $Y_1$, $Y_2$, $Y_3$ identiques ou différents sont choisis parmi Cl, Br et H

– n est un nombre entier égal à 1 ou 2

– au moins l'un des atomes Y situés en α du groupe CO est un halogène choisi parmi Cl et Br

Parmi les anhydrides répondant à la formule (I) on peut citer les anhydrides mono, di, trichloracétiques, mono, di, tribromacétiques, α monochloropropionique, αα dichloropropionique, αα β trichloropropioniques, α monobromopropionique, αα dibromopropionique, ainsi que les anhydrides mixtes du type chlorobromoacétiques ou chlorobromopropioniques.

On préfère cependant utiliser les anhydrides chloroacétiques pour lesquels n = 1 et y est choisi parmi H et Cl car ce sont ceux qui sont les plus facilement disponibles et tout particulièrement l'anhydride dichloracétique car c'est celui qui dans les conditions de réaction est le plus stable thermiquement et le plus économique.

Pour obtenir de meilleurs rendements la réaction est réalisée en présence d'un excès d'acide trifluoracétique par rapport à la stœchiométrie de la réaction. La quantité totale d'acide trifluoracétique est comprise de préférence entre 2 fois et 5 fois la stœchiométrie et tout particulièrement d'environ 4 fois la stœchiométrie.

La réaction est réalisée de préférence à la température d'ébullition du mélange acide trifluoracétique, anhydride d'acide α halogéné et sous pression atmosphérique de manière à distiller l'anhydride trifluoracétique formé. L'anhydride trifluoracétique ayant un point d'ébullition de 39,5° C, il peut être aisément distillé en continu du mélange. Ainsi la température du mélange peut selon un mode de réalisation particulier de l'invention être maintenue entre 80 et 115° C.

Lorsque la distillation est terminée, on monte progressivement la température de manière à récupérer l'acide trifluoracétique en excès dont le point d'ébullition est de 71° C puis l'acide dichloracétique sous produit de la réaction.

Selon un premier mode de mise en œuvre, l'anhydride d'acide α halogéné peut être préparé par réaction d'un halogénure de cet acide avec l'anhydride acétique en présence d'un excès d'halogénure. L'halogénure d'acide est choisi de préférence parmi les chlorures ou les bromures d'acides et tout particulièrement parmi les chlorures d'acides. La réaction est avantageusement facilitée lorsque l'excès d'halogénure d'acide est d'au moins 25% par rapport à la stœchiométrie.

Selon ce premier mode de mise en œuvre de l'invention, on met en contact de préférence dans une première étape l'halogénure de l'acide α halogéné avec de l'anhydride acétique, on sépare le chlorure d'acétyle formé puis l'excès de chlorure d'acide α halogéné, dans une deuxième étape on met en contact le mélange restant avec de l'acide trifluoracétique et on distille l'anhydride trifluoracétique obtenu.

Selon un deuxième mode de mise en œuvre de l'invention, l'anhydride d'acide α halogéné peut être préparé par réaction d'un acide α halogéné avec l'anhydride acétique. Dans ce cas, tout en opérant en présence d'un excès d'acide α halo-

géné, le rendement en anhydride d'acide α halogéné est souvent relativement faible.

Selon ce deuxième mode de mise en œuvre de l'invention, on préfère dans une première étape préparer l'anhydride d'acide α halogéné, comme décrit dans le brevet allemand DE 1 100 612, en présence d'un solvant chimiquement inerte dans les conditions de la réaction, ayant un point d'ébullition supérieur à celui de l'acide acétique ou formant avec l'acide acétique un azéotrope ayant un point d'ébullition inférieur à celui de l'acide acétique.

Ce solvant facilite la distillation continue de l'acide acétique formé ce qui permet d'augmenter les rendements en anhydride d'acide α halogéné. On peut utiliser comme solvant le chlorobenzène et le toluène. On préfère tout particulièrement utiliser le chlorobenzène.

On utilise de préférence une quantité pondérale de solvant par rapport à l'anhydride acétique mis en œuvre supérieure à 500 g par mole d'anhydride et tout particulièrement comprise entre 500 et 2000 g par mole. L'acide α halogéné est mis en œuvre légèrement en excès par rapport à la stœchiométrie et de préférence suivant une quantité comprise entre 1 et 1,1 fois la stœchiométrie.

Dans une deuxième étape, on met en contact le mélange issu de la première étape avec de l'acide trifluoracétique et on distille l'anhydride trifluoracétique obtenu.

L'anhydride trifluoracétique issu du procédé selon l'invention peut être utilisé comme intermédiaire de synthèse dans l'industrie pharmaceutique ou phytosanitaire.

La présente invention sera plus aisément comprise à l'aide des exemples suivants, donnés à titre indicatif mais non limitatif.

*Exemple 1*

Dans un ballon de laboratoire en verre de 1 litre, équipé d'une colonne de distillation à plateaux en verre, de 20 plateaux, on charge 456 g d'acide trifluoracétique technique (contenant 0,3% d'eau au maximum), ce qui correspond sensiblement à 4 moles, puis 120 g, soit 0,5 mole d'anhydride dichloroacétique pur ce qui correspond à un rapport molaire acide trifluoracétique/anhydride dichloracétique de 8. On chauffe le mélange à reflux total, sous pression atmosphérique. La température en tête de colonne s'établit progressivement à 39-39,5° C. Après une heure environ, on soutire le liquide condensé en maintenant un taux de reflux d'environ 20, ceci tant que la température reste à 39-39,5° C. On arrête le soutirage lorsque la température amorce une montée rapide, la fraction étant séparée dès 40° C. On recueille ainsi en 2 heures 30 minutes, 70,5 g de produit identifié en spectrographie infrarouge comme de l'anhydride trifluoracétique $(CF_3CO)_2O$. La masse recueillie représente 0,335 mole, ce qui représente une fraction de l'anhydride dichloracétique initial transformé égale à 0,67.

*Exemple 2*

On répète le mode opératoire de l'exemple 1, avec le même nombre de moles de réactifs, mais en utilisant successivement, à la place de $(CCl_2HCO)_2O$, les anhydrides $(CH_3CO)_2O$, $(CH_2ClCO)_2O$, $(CCl_3CO)_2O$. La fraction, déterminée, comme ci-dessus, de chacun de ces anhydrides transformés en anhydride trifluoracétique, s'établit dans le même ordre, aux valeurs suivantes:

0,36 - 0,59 - 0,74

*Exemple 3*

Dans un ballon de 3 litres surmonté d'une colonne à distiller à plateaux de verre, on introduit 774 g (6 moles) de $CHCl_2COOH$ anhydre et 204 g (2 moles) d'anhydride acétique. On porte la température du mélange à 140° C, puis on distille à une température de 90° C sous pression réduite à la valeur de 300 mmHg l'acide acétique. On sépare ainsi 130 g d'acide acétique soit 2,16 moles. En abaissant progressivement la pression, on distille une fraction essentiellement formée d'anhydride acétique. On abaisse encore la pression à environ 20 mmHg et on distille à la température de 100 à 105° C, en tête de colonne, une fraction de 500 g de $CHCl_2COOH$ (3,88 moles), le restant non distillé correspondant donc à une production d'environ 1,06 mole d'anhydride dichloracétique.

On refroidit le ballon de réaction à environ 50° C, puis on charge 1140 g de $CF_3COOH$ *pur distillé* (10 moles), ce qui correspond à un rapport molaire acide trifluoracétique/anhydride dichloracétique de 9,4 g. On chauffe à reflux. La température en tête de colonne est de 39,5° C, ce qui correspond au point d'ébullition de $(CF_3CO)_2O$. Après environ 5 heures de distillation, on a recueilli 178,5 g de $(CF_3CO)_2O$ (nature chimique vérifiée par analyse par spectrographie RMN de F), soit 0,85 mole. On poursuit la distillation. La température monte à 71° C, correspondant à la distillation de $CF_3COOH$. Lorsque la température atteint 120° C dans le bouilleur et qu'on n'observe plus de reflux, on arrête le chauffage. La fraction de $CF_3COOH$ séparée pèse 911 g (7,99 moles). Le résidu de distillation est composé essentiellement de $CHCl_2COOH$. Le rendement en anhydride trifluoracétique par rapport à l'acide trifluoracétique consommé est de 84,6%.

*Exemple 4*

Dans un ballon de 1 litre avec colonne et équipement nécessaire à la distillation et muni d'une ampoule à brome, on introduit 441 g soit 3 moles de $CHCl_2COCl$. On chauffe jusqu'à 100° C, puis on introduit par l'ampoule à brome 102 g (soit 1 mole) d'anhydride acétique en 1 h 30 mn environ. On poursuit le chauffage et on distille le chlorure d'acétyle vers 50-51° C en tête de colonne. On sépare ainsi 141 g de chlorure d'acétyle (soit 1,796 mole) ce qui correspond à une production d'environ 0,90 mole d'anhydride dichloracétique puis on abaisse la pression et on distille une fraction contenant principalement l'excès de $CHCl_2COCl$ et un peu de chlorure d'acétyle non séparé avec la première fraction. On recueille ainsi 137 g.

On stoppe le chauffage, on refroidit vers 40° C puis on introduit alors 684 g, soit 6 moles de $CF_3COOH$ pur distillé, ce qui correspond à un rapport molaire acide trifluoracétique/anhydride dichloracétique de 6,6. On chauffe à nouveau à reflux. On procède comme à l'exemple précédent pour l'obtention de $(CF_3CO)_2O$ et on recueille 154 g (soit 0,733 mole) de ce produit. On sépare ensuite successivement:

– une fraction de 474 g de $CF_3COOH$ excédentaire (4,15 moles)

– une fraction de 254 g de $CHCl_2COOH$ (1,96 mole).

Il reste un résidu de 33 g. Le rendement en anhydride trifluoracétique par rapport à l'acide trifluoracétique consommé est de 79,2%.

*Exemple 5*

On opère comme pour l'exemple 4.

*Première phase:* pour 1 mole d'anhydride acétique on introduit 3 moles de $CHCl_2COCl$ (excès de 50%). On recueille:

a) 140 g de chlorure d'acétyle, ce qui correspond à un taux de transformation de l'anhydride acétique de 89% en chlorure d'acétyle.

b) 140 g d'excès de $CHCl_2COCl$ contenant un peu de chlorure d'acétyle.

*Deuxième phase:* on introduit 8 moles de $CF_3COOH$ (912 g), ce qui correspond à un rapport molaire acide trifluoracétique/anhydride dichloracétique de 9. On recueille 168 g de $(CF_3CO)_2O$ soit 0,8 mole puis 706 g d'excès de $CF_3COOH$ (6,19 moles) puis un distillat de $CHCl_2COOH$ de 238 g (1,846 mole). Le résidu pèse 33 g.

Le rendement en anhydride trifluoracétique par rapport à l'acide trifluoracétique consommé est de 88%.

*Exemple 6*

On opère comme ci-dessus mais:

*Première phase:* pour 1 mole d'anhydride acétique, on introduit 2,6 moles seulement de $CHCl_2COCl$.

Après réaction on sépare par distillation:

– 134 g (1,707 mole) de chlorure d'acétyle ce qui correspond à un taux de transformation en $(CHCl_2CO)_2O$ de 85,3%, puis

– 101 g contenant essentiellement l'excès de $CHCl_2COCl$, ce qui correspondrait à 0,687 mole.

*Deuxième phase:* introduction de 4 moles de $CF_3COOH$ soit 456 g ce qui correspond à un rapport molaire acide trifluoracétique/anhydride dichloracétique de 4,7. On recueille par distillation en 6 heures 148 g soit 0,704 mole de $(CF_3CO)_2O$, puis 227 g de $CF_3COOH$ (1,99 mole) et 187 g de $CHCl_2COOH$, soit 1,45 mole.

Le résidu est de 45 g.

Le rendement en anhydride trifluoracétique par rapport à l'acide trifluoracétique consommé est de 70%.

*Exemple 7*

Dans un ballon de deux litres, équipé avec une colonne à distiller de 10 plateaux en verre, on introduit 1000 grammes de monochlorobenzène sec, 102 grammes d'anhydride acétique pur (soit 1 mole) et 284 grammes d'acide dichloracétique pur (soit 2,2 moles).

On chauffe le mélange à reflux total et on constate que la température de tête de colonne se stabilise rapidement à 112-113° C. On distille alors à cette température en 7 heures et on obtient 205 grammes d'un mélange; l'analyse indique que la teneur en $CH_3COOH$ de ce mélange est de 52% (106,6 grammes). On distille ensuite en un peu moins d'une heure une fraction passant de 113° C au début à 128° C à la fin, pesant 48 grammes, contenant d'après l'analyse 10,9% de $CH_3COOH$, soit 5,2 grammes.

La quantité totale de $CH_3COOH$ séparé du mélange réactionnel est de 111,8 grammes, ce qui correspond à un taux de conversion de $(CH_3CO)_2O$ de 93% environ.

On refroidit le ballon à environ 40° C, puis on introduit 456 grammes de $CF_3COOH$ pur anhydre (soit 4 moles). Puis on chauffe à reflux. La température dans le bouilleur s'établit à 105° C, celle en tête de colonne se stabilise à 38,5-39,5° C. On distille en 6 heures 152 grammes de $(CF_3CO)_2O$, puis la réaction s'arrête. On refroidit alors, rajoute 456 grammes de $CF_3COOH$ pur anhydre et reprend la distillation de la même façon qu'après la première addition. En fin de distillation, la température du bouilleur atteint 90° C. On distille 52 grammes supplémentaires de $(CF_3CO)_2O$ en environ 2 heures.

La quantité totale d'anhydride trifluoracétique récupérée est donc de 204 grammes, soit 0,97 mole.

On distille ensuite l'excès de $CF_3COOH$ et récupère ainsi 646 grammes de cet acide, soit 5,67 moles. La température au bouilleur est alors de 136° C. La consommation de $CF_3COOH$ s'est donc élevée à (8-5,67) moles soit 2,33 moles et le rendement en $(CF_3CO)_2O$ rapporté à $CF_3COOH$ consommé de 83,4%.

*Exemple 8*

On effectue la première phase de l'opération comme dans l'exemple 7 avec les mêmes quantités de réactifs et de solvant. On recueille une première fraction distillée de 182 grammes contenant 63% d'acide acétique (1,916 mole d'acide acétique).

On recueille une deuxième fraction en continuant la distillation jusqu'à 131° C en tête de colonne.

La quantité totale de $CH_3COOH$ recueillie représente 1,928 mole, ce qui correspond à un taux de conversion de $(CH_3CO)_2O$ de 96,4%.

Après refroidissement, on ajoute en une fois 912 grammes (8 moles) d'acide trifluoroacétique commercial non redistillé et procède comme dans l'exemple 7 pour distiller l'anhydride trifluoroacétique. On recueille successivement:

– 152 grammes, soit 0,723 mole de $(CF_3CO)_2O$

– 758 grammes, dont 741 grammes de $CF_3COOH$ (6,50 moles)

– 18 grammes de monochlorobenzène.

Dans ce premier cycle, la conversion en $(CF_3CO)_2$, rapportée à $(CH_3CO)_2O$ mis en œuvre est donc de 72,3% et le rendement rapporté à $CF_3COOH$ consommé, de 96,4%.

On rajoute au réacteur-bouilleur environ 190 grammes de monochlorobenzène anhydre pour compenser la quantité sortie dans les distillats du premier cycle (1re phase), puis 102 grammes de $(CH_3CO)_2O$ pur (1 mole).

On opère comme pour le premier cycle et obtient 2 fractions distillées, la première de 175 grammes contenant 104 grammes de $CH_3COOH$, soit 1,733 mole, la seconde de 100 grammes, contenant 11 grammes de $CH_3COOH$, soit 0,183 mole.

La totalité de $CH_3COOH$ sorti du système représente donc 1,916 mole.

Pour réaliser la seconde phase de l'opération, on ajoute 735 grammes pris dans le lot de 758 g contenant l'excès de $CF_3COOH$ du cycle précédent, ce qui correspond à 6,3 moles et 193,5 grammes de $CF_3COOH$ pris dans un lot de produit commercial, soit 1,7 mole. Puis on poursuit comme décrit précédemment. On sépare alors par distillation.

– 189 grammes, soit 0,90 mole de $(CF_3CO)_2O$

– 701 grammes, soit 6,14 moles de $CF_3COOH$

Au cours de ce deuxième cycle, la conversion en $(CF_3CO)_2O$ recueilli, rapportée à $(CH_3CO)_2O$ mis en jeu, représente 90%, son rendement rapporté à $CF_3COOH$ transformé, 96,7%.

*Exemple 9*

On opère comme à l'exemple 7 en remplaçant l'acide dichloroacétique par 239 grammes (soit 2,2 moles) d'acide α chloropropionique. On chauffe à reflux et la température de tête se stabilise à 112-113° C. On distille alors et recueille en 6 heures 197 grammes de liquide dont on extrait 111 grammes de $CH_3COOH$. On recueille ensuite une fraction de 51 grammes (Arrêt à 130° C en tête) contenant, par dosage, 8 grammes de $CH_3COOH$.

Après refroidissement à 70° C environ du liquide restant dans le ballon, on introduit 912 grammes de $CF_3COOH$ anhydre, soit 8 moles.

On opère comme dans les autres exemples et on recueille en 7 heures 95 grammes de $(CF_3CO)_2O$ passant à 39,5° C; on sépare ensuite une fraction de 34 grammes de ce même constituant, en 5 heures supplémentaires. La quantité totale d'anhydride trifluoracétique récupéré est de 129 g (0,614 m). On récupère ensuite 750 g de $CF_3COOH$ (6,58 moles). La consommation de $CF_3COOH$ s'est donc élevée à (8-6,58 moles) soit 1,42 mole et le rendement en $(CF_3CO)_2O$ rapporté à $CF_3COOH$ consommé, de 86%.

## Revendications

1. Procédé de préparation d'anhydride trifluoracétique, caractérisé par le fait que l'on fait réagir l'acide trifluoracétique avec l'anhydride d'un acide α halogéné.

2. Procédé selon la revendication 1, caractérisé par le fait que l'anhydride de l'acide α halogéné répond à la formule générale (I):

$$[Y_1(CY_2Y3)_n-CO]_2O \qquad (I)$$

dans laquelle:

– $Y_1$, $Y_2$, $Y_3$ identiques ou différents sont choisis parmi Cl, Br, et H

– n est un nombre entier égal à 1 ou 2

– au moins l'un des Y situé en α du CO doit être Cl ou Br.

3. Procédé selon la revendication 2, caractérisé par le fait que l'anhydride répond à la formule générale (I) dans laquelle n = 1 et Y est choisi parmi Cl et H.

4. Procédé selon la revendication 3, caractérisé par le fait que l'anhydride est l'anhydride dichloracétique.

5. Procédé selon la revendication 1, caractérisé par le fait que l'acide trifluoracétique est mis en excès molaire par rapport à la stœchiométrie de la réaction.

6. Procédé selon la revendication 5, caractérisé par le fait que la quantité molaire d'acide trifluoracétique utilisé est comprise entre 2 et 5 fois la stœchiométrie de la réaction et de préférence est égale à environ 4 fois la stœchiométrie.

7. Procédé selon la revendication 1, caractérisé par le fait que la réaction est réalisée à une température supérieure au point d'ébullition du mélange mis en œuvre.

8. Procédé selon la revendication 1 et la revendication 3, caractérisé par le fait que la température de réaction est comprise entre 80° C et 115° C.

9. Procédé selon la revendication 1, caractérisé par le fait que la réaction est réalisée à la pression atmosphérique.

10. Procédé selon l'une quelconque des revendications 1 à 4, dans lequel l'anhydride d'acide α halogéné est obtenu par réaction d'un halogénure dudit acide halogéné sur l'anhydride acétique.

11. Procédé de préparation d'anhydride trifluoracétique, caractérisé par le fait que dans une première étape on met en contact l'halogénure d'un acide α halogéné avec de l'anhydride acétique, on sépare le chlorure d'acétyle formé puis l'excès de chlorure d'acide α halogéné, dans une deuxième étape on fait réagir le mélange restant avec l'acide trifluoracétique.

12. Procédé de préparation d'anhydride trifluoracétique, caractérisé par le fait que dans une première étape, on met en contact l'acide α halogéné avec de l'anhydride acétique en présence de monochlorobenzène, on sépare l'azéotrope formé entre l'acide acétique et le monochlorobenzène, dans une deuxième étape, on fait réagir le mélange restant avec l'acide trifluoracétique.

## Patentansprüche

1. Verfahren zur Herstellung von Trifluoracetanhydrid, dadurch gekennzeichnet, dass man Trifluoressigsäure mit dem Anhydrid einer α-Halogensäure reagieren lässt.

2. Verfahren nach Anspruch 1, dadurch gekennzeichnet, dass das Anhydrid der α-Halogensäure der nachfolgenden allgemeinen Formel (I):

$$[Y_1(CY_2Y_3)_n{-}CO]_2O \qquad (I)$$

entspricht, in der:

– $Y_1$, $Y_2$, $Y_3$ identisch oder unterschiedlich sind und ausgewählt werden zwischen Cl, Br und H

– n eine ganze Zahl und gleich 1 oder 2 ist

– mindestens eines der Atome Y in α-Stellung der CO-Gruppe, Cl oder Br ist.

3. Verfahren nach Anspruch 2, dadurch gekennzeichnet, dass das Anhydrid der allgemeinen Formel (I) entspricht, in der n gleich 1 und Y ausgewählt wird aus Cl und H.

4. Verfahren nach Anspruch 3, dadurch gekennzeichnet, dass das Anhydrid Dichloracetanhydrid ist.

5. Verfahren nach Anspruch 1, dadurch gekennzeichnet, dass die Trifluoressigsäure in bezug auf die Stöchiometrie der Reaktion in molarem Überschuss eingesetzt wird.

6. Verfahren nach Anspruch 5, dadurch gekennzeichnet, dass die eingesetzte molare Menge der Trifluoressigsäure dem 2 bis 5-fachen der stöchiometrischen Menge, vorzugsweise dem 4-fachen der stöchiometrischen Menge entspricht.

7. Verfahren nach Anspruch 1, dadurch gekennzeichnet, dass die Reaktion bei einer Temperatur oberhalb des Siedepunkts der eingesetzten Mischung durchgeführt wird.

8. Verfahren nach Anspruch 1 und Anspruch 3, dadurch gekennzeichnet, dass die Reaktionstemperatur zwischen 80° C und 115° C liegt.

9. Verfahren nach Anspruch 1, dadurch gekennzeichnet, dass die Reaktion bei Atmosphärendruck durchgeführt wird.

10. Verfahren nach einem der Ansprüche 1 bis 4, bei dem das Anhydrid der α-Halogensäure durch Reaktion eines Halogenids dieser halogenierten Säure mit Acetanhydrid erhalten wird.

11. Verfahren zur Herstellung von Trifluoracetanhydrid, dadurch gekennzeichnet, dass man in einem ersten Schritt das Halogenid einer α-halogenierten Säure mit Acetanhydrid in Kontakt bringt, das gebildete Acetylchlorid entfernt, dann den Überschuss des Chlorids der α-halogenierten Säure entfernt und in einem zweiten Schritt die verbleibende Mischung mit Trifluoressigsäure reagieren lässt.

12. Verfahren zur Herstellung von Trifluoracetanhydrid, dadurch gekennzeichnet, dass man in einem ersten Schritt eine α-Halogensäure in Gegenwart von Monochlorbenzol mit Acetanhydrid in Kontakt bringt, das zwischen Essigsäure und dem Monochlorbenzol gebildete Azeotrop abtrennt und in einem zweiten Schritt die verbleibende Mischung mit Trifluoressigsäure reagieren lässt.

**Claims**

1. Process for the preparation of trifluoroacetic anhydride, characterized in that trifluoroacetic acid is reacted with the anhydride of an α-halogenated acid.

2. Process according to Claim 1, characterized in that the anhydride of the α-halogenated acid corresponds to the general formula (I):

$$[Y_1\ (CY_2Y_3)_n{-}CO]_2O \qquad (I)$$

in which $Y_1$, $Y_2$ and $Y_3$, which may be identical or different, are chosen from amongst Cl, Br and H, n is an integer equal to 1 or 2, and at least one of the Y's located in the α-position to the CO must be Cl or Br.

3. Process according to Claim 2, characterized in that the anhydride corresponds to the general formula (I), in which n = 1 and Y is chosen from amongst Cl and H.

4. Process according to Claim 3, characterized in that the anhydride is dichloroacetic anhydride.

5. Process according to Claim 1, characterized in that the trifluoroacetic acid is used in molar excess relative to the stoichiometric amount for the reaction.

6. Process according to Claim 5, characterized in that the molar amount of trifluoracetic acid used is between 2 and 5 times the stoichiometric amount for the reaction and preferably equal to about 4 times the stoichiometric amount.

7. Process according to Claim 1, characterized in that the reaction is carried out at a temperature above the boiling point of the mixture employed.

8. Process according to Claim 1 and Claim 3, characterized in that the reaction temperature is between 80° C and 115° C.

9. Process according to Claim 1, characterized in that the reaction is carried out at atmospheric pressure.

10. Process according to any one of Claims 1 to 4, in which the anhydride of the α-halogenated acid is obtained by reaction of a halide of the said halogenated acid with acetic anhydride.

11. Process for the preparation of trifluoroacetic anhydride, characterized in that in a first stage the halide of an α-halogenated acid is brought into contact with acetic anhydride, and the acetyl chloride formed, and then the excess of the chloride of the α-halogenated acid, are removed, and in a second stage the residual mixture is reacted with trifluoroacetic acid.

12. Process for the preparation of trifluoroacetic anhydride, characterized in that in a first stage the α-halogenated acid is brought into contact with acetic anhydride in the presence of monochlorobenzene, the azeotrope formed between acetic acid and monochlorobenzene is removed, and in a second stage the residual mixture is reacted with trifluoroacetic acid.